## Europäisches Patentamt
## European Patent Office
## Office européen des brevets

(19)

(11) Veröffentlichungsnummer: **0 343 405**
**A1**

(12) **EUROPÄISCHE PATENTANMELDUNG**

(21) Anmeldenummer: 89108040.0

(22) Anmeldetag: 03.05.89

(51) Int. Cl.⁴: **A61B 5/00**

(30) Priorität: 19.05.88 DE 3817052

(43) Veröffentlichungstag der Anmeldung:
**29.11.89 Patentblatt 89/48**

(84) Benannte Vertragsstaaten:
**DE FR GB SE**

(71) Anmelder: **Drägerwerk Aktiengesellschaft**
**Moislinger Allee 53-55**
**D-2400 Lübeck 1(DE)**

(72) Erfinder: **Friesdorf, Wolfgang, Dr.**
**Auf der Breite 8**
**D-7907 Langenau(DE)**
Erfinder: **Ryschka, Martin, Dr. rer. nat.**
**Morierstrasse 10b**
**D-2406 Stockelsdorf(DE)**
Erfinder: **Bayerlein, Jörg, Dr.**
**Schulweg 4d**
**D-2406 Stockelsdorf(DE)**

(54) Verfahren zur überwachung von Patientendaten und Schaltungsanordnung hierzu.

(57) Bei der Überwachung von Patienten werden die Patientendaten als einzelnen Organsystemen zugeordnete Parameterwerte durch entsprechende Sensoren (1...5) abgetastet. Zur optimalen Darstellung eines Datenfeldes, das mehrere Parameter umfasst, werden mit Hilfe eines Netz-Schaltwerks (8,23,24) organsystembezogene Übersichtsschaubilder gebildet, welche durch Abrufbefehl ausgegeben werden.

Fig.1

**EP 0 343 405 A1**

## Verfahren zur Überwachung von Patientendaten und Schaltungsanordnung hierzu

Die Erfindung betrifft ein Verfahren zur Überwachung von Patientendaten, die als Parameterwerte einzelnen Organen bzw. Organsystemen zugeordnet werden können, und die durch entsprechende Sensoren ermittelt werden. Außerdem wird eine vorteilhafte Schaltungsanordnung zur Durchführung des Verfahrens angegeben.

Die Überwachung von Patientendaten z.B. Daten der Lunge oder des Herz/Kreislaufsystems ist in vielen Ausführungsformen durch anzeigende Überwachungsgeräte bekannt.

Zum bekannten Stande der Technik gehört ferner ein Narkosesimulator, wie er in der Literaturstelle "Abbott -Narkosesimulator" von Helmut Schwilden (Deutsche Abbott GmbH, Dezember 1986) beschrieben ist. Dort wird mit Hilfe eines Rechners die mögliche Verdampfereinstellung des Narkosegerätes mit den Parametern des Beatmungssystems und gewissen physiologischen Größen des Patienten verknüpft. Nach Einstellung der für die Narkose wesentlichen Daten des Patienten und des Narkosegerätes sowie nach Wahl des Narkosemittels wird der Prozentsatz des Herzzeitvolumens angezeigt, mit dem die einzelnen Organe perfundiert werden. Jedem Organ sind zwei Größen zugeordnet, nämlich der prozentuale Anteil des Organs am Gesamtkörpervolumen und der Prozentsatz des Herzzeitvolumens, mit dem dieses Organ perfundiert wird. Es handelt sich somit um die Aufteilung eines als Patientenwert ermittelten Meßparameters, z.B. des Herzminutenvolumens auf die wichtigsten Organe und um die bildmäßige Darstellung dieser Aufteilung. Dabei werden aus wenigen Patientendaten durch Aufteilung eine Mehrzahl von Teildaten der gleichen Meßqualität erzeugt.

Die Erfindung geht von der Aufgabenstellung aus, eine Mehrzahl von Patientendaten zu ordnen und anzuzeigen oder darzustellen, und zwar derart, daß eine dem Patienten sowie dem behandelnden Arzt optimal angepaßte, vorher festgelegte Anzeigeform entsteht. Der Vorteil einer solchen festgelegten Anzeigeform liegt darin, daß anstelle einer fast unübersehbaren Fülle von Daten, wie sie beispielsweise bei der Intensivpflege von Patienten überwacht und angezeigt werden, eine Datensortierung nach medizinischen Gesichtspunkten vorgenommen wird, welche einen optimalen Überblick ermöglicht, so daß der aktuelle Zustand des Patienten schneller und sicherer beurteilt werden kann.

Die Lösung dieser Aufgabenstellung erfolgt dadurch, daß aus einem eine Mehrzahl von Parameterwerten enthaltenden Datenfeld mit Hilfe eines Netz-Schaltwerks organsystembezogene Übersichtsschaubilder gebildet und durch Abrufbefehl ausgegeben werden.

Die Anzeige der organsystembezogenen Parameterwerte kann dabei zweckmäßig kurvenförmig als Funktion ihres zeitlichen Verlaufs erfolgen. Aus dem zeitlichen Verlauf der Parameterwerte läßt sich gegebenenfalls vorteilhaft eine den Trend repräsentierende Anzeigegröße bestimmen.

In weiterer Ausgestaltung der Erfindung kann vorgesehen sein, daß aus dem Verlauf der organsystembezogenen Parameter Alarmmeldungen abgeleitet werden. Diese Alarmmeldungen lassen sich zweckmäßig nach Dringlichkeitsstufen unterteilen und durch unterschiedliche Anzeigen bzw. Anzeigemittel wahrnehmbar machen.

Die organsystembezogenen Übersichtsschaubilder können zweckmäßig so ausgewählt werden, daß sie den aktuellen Zustand des Patienten zum Zeitpunkt der Abfrage anzeigen. In Verbindung mit vorhandenen Speicherelementen läßt sich jedoch auch eine Abfrage in der Weise festlegen, daß zum Zeitpunkt der Abfrage ein Überblick über eine vorangehende Zeitspanne angezeigt wird. Diese Zeitspanne kann mehrere Stunden oder Tage betragen.

In der praktischen Ausbildung erscheint es zweckmäßig, wenigstens Übersichtsschaubilder des Bewußtseins/ZNS, des Atmungssystems, des Herz/Kreislaufsystems und des Stoffwechselsystems mit zugehörigen Parametern zu erzeugen. Im einzelnen richtet sich die Einteilung der organsystembezogenen Übersichtsschaubilder nach der klinischen Beurteilungspraxis, die jedoch entsprechend den Wünschen des behandelnden Arztes modifiziert werden kann. Es erscheint ferner zweckmäßig, den Therapieplan kennzeichnende Werte zu speichern und auf Abruf organsystembezogen auszugeben.

Eine zweckmäßige Ausbildung kann vorsehen, daß die Parameterwerte in der Speichereinheit des Computers in mehreren Ablagefeldern gespeichert werden, und daß in Verbindung mit der Speichereinheit Auswahlbefehle z.B. zur Auswahl von Organsystemen und zur Auswahl der Behandlungsgrößen möglich sind.

In der Zeichnung wird das erfindungsgemäße Verfahren anhand einer Schaltungsanordnung erläutert, aus der sich weitere Erfindungsmerkmale ergeben; es zeigen:

Fig. 1 eine Schaltungsanordnung in Blockschaltbilddarstellung,

Fig. 2 ein mit der Schaltung nach Fig. 1 angezeigtes Übersichtsschaubild des Herz/Kreislaufsystems,

Fig. 3 ein mit der Schaltungsanordnung nach Fig. 1 erzeugtes Übersichtsbild als Auszug aus verschiedenen organsystembezogenen Übersichtsschaubildern.

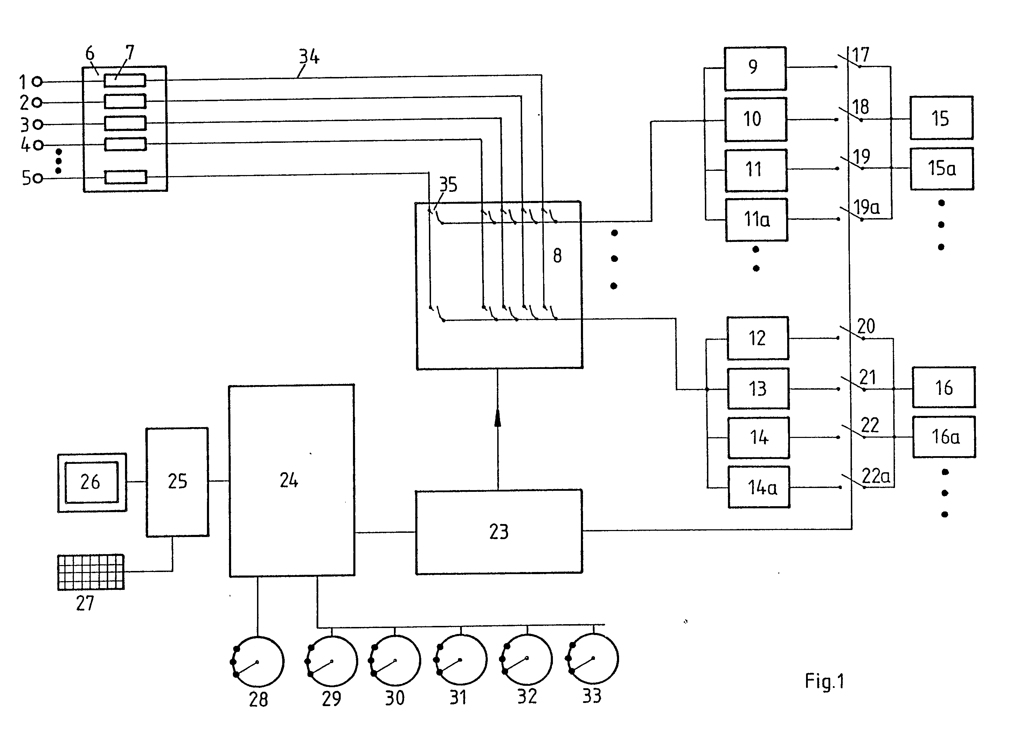

Die schematische Schaltungsanordnung nach Fig. 1 dient dazu, beispielhaft die von fünf Sensoren 1-5 abgetasteten Meßwerte auf Displayfeldern 15,16 in einzelnen organsystembezogenen Übersichtsschaubildern so anzuzeigen und/oder darzustellen, daß eine dem Patientenzustand sowie dem Arzt optimal angepaßte vorher festgelegte Anzeigeform entsteht.

Die Signale aus den Sensoren 1-5, welche beispielsweise Blutdrücke, Herzrate, Sauerstoffsättigung, endexpiratorisches $CO_2$, Temperatur und dergleichen überwachen, werden in eine Trendspeichereinheit 6 eingeführt, in der für jedes Signal ein Trendspeicher 7 vorgesehen ist. Die Signale werden in einem festen Zeittakt abgetastet und gespeichert. Die Trendspeicher 7 können ausgelesen werden und ihre Daten gelangen über Verbindungsleitungen, z.B. 34 zu einer Schaltmatrix 8. Dort kann aufgrund der Schalterstellung einer Mehrzahl von Parameterauswahlschaltern 35 eine Auswahl getroffen werden, welche Daten aus den Trendspeichern 7 an die weiterverarbeitenden Displayprozessoren 9-14 gelangen sollen. Die Displayprozessoren 9-14 bereiten die Inhalte der Trendspeicher 7 für die gewünschte Anzeigeform auf und leiten sie den in beliebiger Anzahl möglichen Displayfeldern 15,16 zu.

So kann beispielsweise der Displayprozessor 9 aus einem Trendinhalt des Trendspeichern 7 eine Trendkurve auf dem Displayfeld 15 erzeugen. Der Displayprozessor 10 kann eine Tabelle wiedergeben, in der die Zahlenwerte abgebildet werden. Der Displayprozessor 11 schließlich kann eine weitere gewünschte organsystembezogene Darstellungsform erzeugen. Über die Zuordnungsschalter 17-19 läßt sich dann auswählen, welcher Displayprozessor auf das entsprechende Displayfeld, z.B. 15 aufgeschaltet wird. So kann beispielsweise mit Hilfe der Schalterkombinationen der Schaltmatrix 8 in Verbindung mit den Zuordnungsschaltern 17-22;19a,22a jeder Trendspeicherinhalt jedes Sensors in der gewünschten Anzeigeart auf jedem Displayfeld 15,16;15a,16a dargestellt werden.

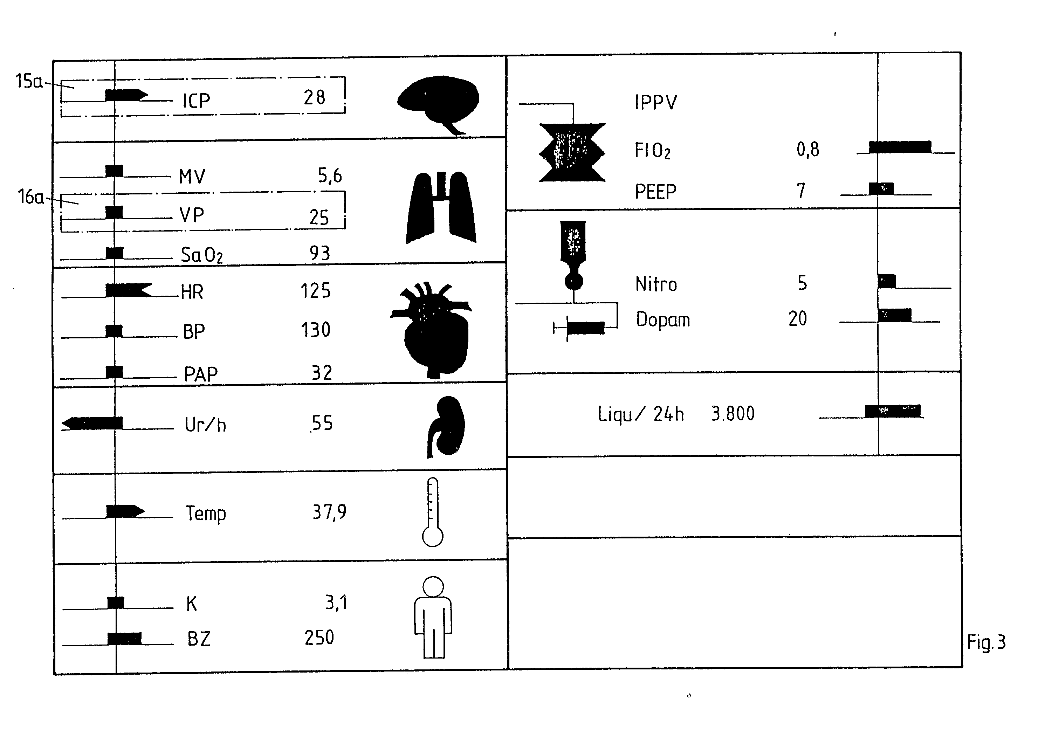

Es können ferner Bildprozessoren 11a,14a durch geeig nete Belegung der Schaltmatrix 8 so mit Patientendaten gespeist werden, daß die Displayfelder 15a,16a jeweils Auszüge der organsystembezogenen Patientendaten enthalten, so daß aus der Zusammenstellung dieser Displayfelder 15a,16a eine Gesamtübersicht des Patientenzustandes entsteht, wie sie beispielsweise in Fig 3 wiedergegeben wird.

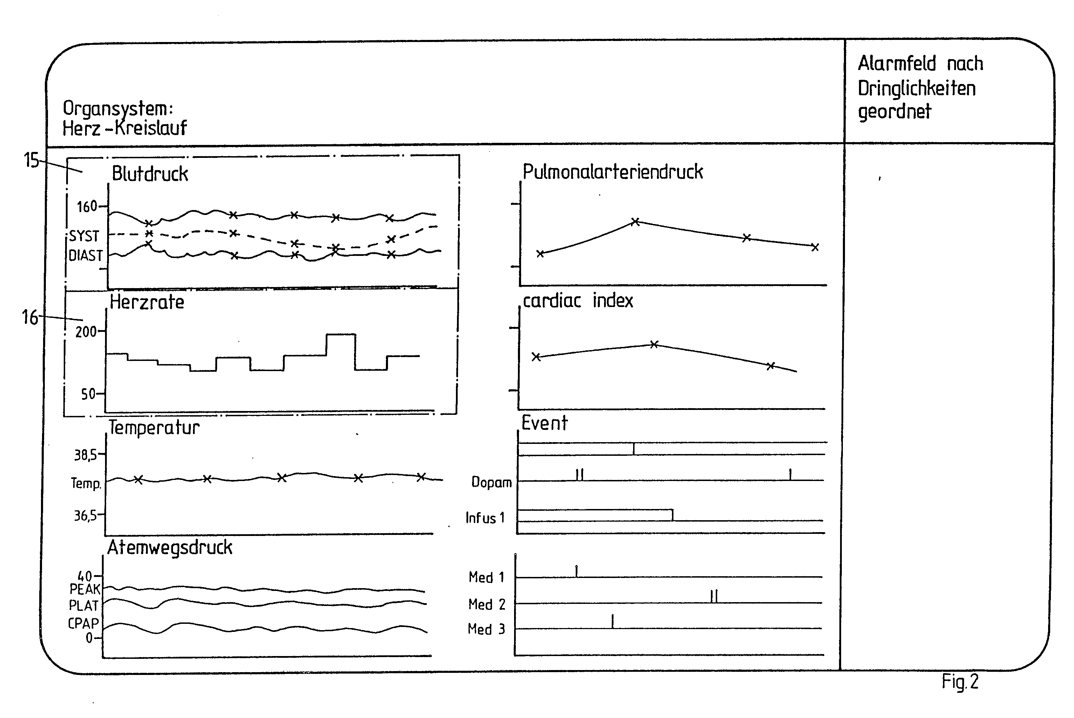

In einem Speicherfeld 24 sind zusätzlich eine große Anzahl von festen Schalterkombinationen der Schaltmatrix 8 abgespeichert. Jede einzelne dieser Schalterkombinationen läßt sich in den Arbeitsspeicher 23 laden, dessen Speicherausgänge sowohl die Eingänge der Schaltmatrix 8 als auch direkt die Zuordnungsschalter 17-22 beeinflussen. Je nach dem Inhalt dieser Arbeitsspeicher 23 wird in den Displayfeldern 15,16 ein Übersichtsschaubild ausgegeben, das eine organsystembezogene Auswahl aus den Sensorsignalen, beispielsweise für das Herz/Kreislaufsystem (vgl. Fig. 2) bildet. Mit Hilfe von Bildauswahlschaltern 28-33 kann aus der großen Anzahl der Schalterkombination des Speicherfeldes 24 eine ganz bestimmte Kombination ausgewählt und in den Arbeitsspeicher 23 geladen werden.

Die Bildauswahlschalter 28-33 lassen sich nach Wunsch frei belegen.

Mit dem Bildauswahlschalter 28 sind verschiedene Organsystembilder anwählbar. Es kann beispielsweise das Herz/Kreislauf-Übersichtsschaubild, das Respirations-Übersichtsschaubild, das Wasser-Elektrolythaushalts-Schaubild usw. eingestellt werden, wo bei die zu den einzelnen Übersichtsschaubildern vorbestimmt ausgewählten Parameterwerte gleichzeitig angezeigt werden.

Die Auswahl der Information wird unter den Bildauswahlschaltern aufgeteilt.

Mit dem Bildauswahlschalter 29 kann eine typische Diagnose z.B. Multitrauma, Bauch-OP oder Herzinfarkt vorgewählt werden. Der Bildauswahlschalter 30 ermöglicht eine Auswahl nach den einzelnen Liegetagen, wobei beispielsweise in Stellung 1 der erste Liegetag, Stellung 2, 2.-4. Liegetag, Stellung 3, 5.-20. Liegetag angezeigt wird, da die für den Arzt wichtige Information auch vom Liegetag abhängen kann.

Der Bildauswahlschalter 31 ermöglicht die Auswahl einer typischen für die Klinik spezifischen Konfiguration, beispielsweise die Kombination verschiedener Behandlungsarten. Diese Klinikkonfiguration ist für einen bestimmten Anwendungsort wesentlich, kann aber von Klinik zu Klinik variiert werden. Mit dem Bildauswahlschalter 32 lassen sich schließlich verschiedene Präferenzen in Parameterkombinationen nach der Auswahl der Bedienungsperson, beispielsweise der aufsichtsführenden Chefärzte berücksichtigen. Auf diese Weise kann auch den individuellen Wünschen des Anwenders entsprochen werden.

Mit dem Bildauswahlschalter 33 kann zuletzt auch noch die patiententypische Abhängigkeit berücksichtigt werden, welche gegebenenfalls ein Kriterium für die Informationsauswahl darstellt. Wichtige Variablen sind dabei Geschlecht, Alter, Körpergewicht und dergleichen.

Die Variablen der Bildauswahlschalter 29-33 können gegebenenfalls vorteilhaft auch automatisch ausgewählt werden, wenn die Bildauswahlschalter mit einem Rechnersystem verbunden sind, das über administrative Daten (Daten aus der persönlichen Krankenakte) verfügt, denn alle Parameter sind Inhalt dieser Krankenakte.

Der Inhalt der großen Anzahl der Speicherkombinationen des Speicherfeldes 24 kann gegebenenfalls vorteilhaft fest programmiert sein (z.B. als Ergebnis einer wissenschaftlichen Studie), läßt sich aber bei Bedarf auch jederzeit vom Anwender selbst verändern. Eine solche Veränderung kann mit einem angeschlossenen Rechner 25 durchgeführt werden, welcher mit einer Bildschirmeinheit 26 und mit einer Tastatur 27 in Verbindung steht. Damit läßt sich jede Speicherstelle des Speicherfeldes 24 aufrufen, gegebenenfalls modifizieren und erneut abspeichern.

Auf diese Weise wird eine große Flexibilität erreicht, die es einerseits ermöglicht, ohne großen Aufwand nur durch Betätigung eines Bildauswahlschalters z.B. 28 für einen Patienten eine geeignete Informationsauswahl zu treffen, andererseits aber läßt sich diese Auswahl an die verschiedenen übergeordneten Gesichtspunkte anpassen.

Obwohl die Ausgabe der Daten über Displayfelder bevorzugt wird, kann für einzelne Anwendungsfälle auch die Ausgabe der gespeicherten oder aktuellen organsystembezogenen Parameterwerte durch einen Drucker zweckmäßig sein.

**Ansprüche**

1. Verfahren zur Überwachung von Patientendaten, die als Parameterwerte einzelnen Organen bzw. Organsystemen zugeordnet werden können, und die durch entsprechende Sensoren ermittelt werden, **dadurch gekennzeichnet,** daß aus einem eine Mehrzahl von Parameterwerten enthaltenden Datenfeld mit Hilfe eines Netz-Schaltwerks organsystembezogene Übersichtsschaubilder gebildet und durch Abrufbefehl ausgegeben werden.

2. Verfahren nach Anspruch 1, **dadurch gekennzeichnet,** daß die Anzeige der organsystembezogenen Parameterwerte kurvenförmig als Funktion ihres zeitlichen Verlaufs erfolgt.

3. Verfahren nach Anspruch 1, **dadurch gekennzeichnet,** daß aus dem zeitlichen Verlauf der Parameterwerte eine den Trend repräsentierende Anzeigegröße bestimmt wird.

4. Verfahren nach Anspruch 1, **dadurch gekennzeichnet,** daß aus dem Verlauf der organbezogenen Parameter Alarmmeldungen abgeleitet werden.

5. Verfahren nach Anspruch 4, **dadurch gekennzeichnet,** daß die Alarmmeldungen nach Dringlichkeitsstufen unterteilt und durch unterschiedliche Anzeigen organsystembezogen mitgeteilt werden.

6. Verfahren nach Anspruch 1, **dadurch gekennzeichnet,** daß die organsystembezogenen Übersichtsschaubilder den aktuellen Zustand zum Zeitpunkt der Abfrage anzeigen.

7. Verfahren nach Anspruch 1, **dadurch gekennzeichnet,** daß die organsystembezogenen Übersichtsschaubilder zum Zeitpunkt der Abfrage einen Überblick über eine vorangehende festgelegte Zeitspanne anzeigen.

8. Verfahren nach Anspruch 7, **dadurch gekennzeichnet,** daß die Zeitspanne mehrere Stunden beträgt.

9. Verfahren nach Anspruch 1, **dadurch gekennzeichnet,** daß wenigstens Übersichtsschaubilder des Bewußtseins/ZNS, des Atemsystems, des Herz/Kreislaufsystems und des Stoffwechselsystems mit zugeordneten Parametern erzeugt werden.

10. Verfahren nach Anspruch 4, **dadurch gekennzeichnet,** daß zusätzlich patientenbezogene Vorgabewerte bei der Generierung der Alarmmeldungen berücksichtigt werden.

11. Verfahren nach Anspruch 1, **dadurch gekennzeichnet,** daß die organsystembezogenen Parameterwerte gespeichert werden.

12. Verfahren nach Anspruch 1, **dadurch gekennzeichnet,** daß Auszüge aus den organsystembezogenen Parameterwerten zu einem Patientenstatus zusammengefaßt werden.

13. Verfahren nach Anspruch 1, **dadurch gekennzeichnet,** daß den Therapieplan kennzeichnende Werte zusätzlich gespeichert und organsystembezogen ausgegeben werden.

14. Schaltungsanordnung, insbesondere zur Durchführung des Verfahrens nach Anspruch 1, **dadurch gekennzeichnet,** daß Sensoren (1-5) mit zugeordneten Trendspeichern (7) verbunden sind, welche mit einer Schaltmatrix (8) in Verbindung stehen, und daß die Ausgänge der Schaltmatrix (8) mit jeweils zugeordneten Displayprozessoren (9,10,11 und 12,13,14) verbunden sind, welche über Zuordnungsschalter (17,18,19 und 20,21,22) mit Displayfeldern (15,16) in Verbindung stehen.

15. Schaltungsanordnung nach Anspruch 14, **dadurch gekennzeichnet,** daß Bildauswahlschalter (28-33) in Verbindung mit einem Speicherfeld (24) vorgesehen sind und daß der Ausgang des Speicherfeldes (24) über einen Arbeitsspeicher (23) mit der Schaltmatrix (8) und mit den Zuordnungsschaltern (17-22) in Verbindung steht.

16. Schaltungsanordnung nach Anspruch 15, **dadurch gekennzeichnet,** daß das Speicherfeld (24) mit einem Rechner (25) in Verbindung steht, der über eine Tastatur (27) und eine Bildschirmeinheit (26) eine Veränderung des Inhaltes des Speicherfeldes (24) herbeiführt.

Fig.1

Organsystem:
Herz–Kreislauf

Alarmfeld nach
Dringlichkeiten
geordnet

Blutdruck

Pulmonalarteriendruck

Herzrate

cardiac index

Temperatur

Event

Atemwegsdruck

EP 0 343 405 A1

Fig. 2

| | | |
|---|---|---|
| ICP | 28 | |
| MV | 5,6 | |
| VP | 25 | |
| SaO$_2$ | 93 | |
| HR | 125 | |
| BP | 130 | |
| PAP | 32 | |
| Ur/h | 55 | |
| Temp | 37,9 | |
| K | 3,1 | |
| BZ | 250 | |

| | | |
|---|---|---|
| IPPV | | |
| FIO$_2$ | 0,8 | |
| PEEP | 7 | |
| Nitro | 5 | |
| Dopam | 20 | |
| Liqu / 24h | 3.800 | |

Fig. 3

EP 0 343 405 A1

Europäisches Patentamt

**EUROPÄISCHER RECHERCHENBERICHT**

Nummer der Anmeldung

EP 89 10 8040

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-A-2 348 582 (GENERAL ELECTRIC CO.) * Seite 5, Zeile 19 - Seite 8, Zeile 20; Seite 13, Zeile 5 - Seite 14, Zeile 29; Seite 22, Zeilen 2-28; Seite 26, Zeile 8 - Seite 27, Zeile 17; Seite 29, Zeile 26 - Seite 31, Zeile 12; Seite 35, Zeilen 6-24; Figuren 1-5,16,17 * | 1-4,6,9 -12,14, 15 | A 61 B 5/00 |
| X | DE-A-2 451 699 (AMERICAN OPTICAL CO.) * Seite 1, Zeilen 1-12; Seite 5, Zeilen 3-18; Seite 7, Zeile 23 - Seite 8, Zeile 30; Seite 9, Zeile 24 - Seite 11, Zeile 16; Figuren 1,2 * | 1-3,6-9 | |
| X | COMPUTERS IN CARDIOLOGY, September 1979, Seiten 461-464, IEEE, New York, US; D. KALINSKY et al.: "The "solo monitor" microcomputer bedside monitor" * Seiten 461-463 * | 1-14,16 | |
| A | US-A-3 814 082 (D.E.M. TAYLOR) * Zusamenfassung; Spalte 1, Zeilen 30-39; Spalte 2, Zeile 65 - Spale 3, Zeile 35; Spalte 4, Zeile 14 - Spalte 5, Zeile 62; Figuren 1-6 * | 3,4,11, 14 | RECHERCHIERTE SACHGEBIETE (Int. Cl.4) A 61 B |
| A | US-A-3 618 592 (J.S.S. STEWART) * Zusammenfassung; Spalte 1, Zeilen 39-47; Spalte 1, Zeile 57 - Spalte 2, Zeile 4; Spalte 2, Zeile 33 - Spalte 4, Zeile 54; Figuren 1-3 * | 1,4-6,9 -12,14 | |

Der vorliegende Recherchenbericht wurde für alle Patentansprüche erstellt

| Recherchenort | Abschlußdatum der Recherche | Prüfer |
|---|---|---|
| DEN HAAG | 24-08-1989 | RIEB K.D. |